# EUROPEAN PATENT SPECIFICATION

(11) **EP 2 809 320 B1**
(45) Date of publication and mention of the grant of the patent: **22.03.2017**
(21) Application number: 13701807.3
(22) Date of filing: 01.02.2013
(51) Int. Cl.: A61K 31/407, A61P 21/02

(54) **(1R,4R)-6'-FLUORO-(N-METHYL- OR N,N-DIMETHYL-)-4-PHENYL-4',9'-DIHYDRO-3'H-SPIRO-[CYCLOHEXANE-1,1'-PYRANO[3,4,B]INDOL]-4-AMINE FOR TREATING FIBROMYALGIA AND CHRONIC FATIGUE SYNDROME**
(1R,4R)-6'-FLUOR-(N-METHYL- ODER -N,N-DIMETHYL)-4-PHENYL-4',9'-DIHYDRO-3'H-SPIRO[CYCLOHEXAN-1,1'-PYRANO-[3,4,B]INDOL-4-AMIN ZUR BEHANDLUNG VON FIBROMYALGIE UND DES CHRONISCHEN ERSCHÖPFUNGSSYNDROMS
(1R,4R)-6'-FLUORO-(N-MÉTHYL- OU N,N-DIMÉTHYL-)-4-PHÉNYL-4',9'-DIHYDRO-3'H-SPIRO-(CYCLOHEXAME-1,1'-PYRANO[3,4,B]INDOL]-4-AMINE POUR TRAITER LA FIBROMYALGIE ET LE SYNDROME DE FATIGUE CHRONIQUE

(30) Priority: 03.02.2012 EP 12000743
(43) Date of publication of application: 10.12.2014
(73) Proprietor: Grünenthal GmbH, 52078 Aachen (DE)
(72) Inventor: FROSCH, Stefanie, 52078 Aachen (DE); LINZ, Klaus, 53359 Rheinbach (DE); BLOMS-FUNKE, Petra, 52146 Würselen (DE)
(86) International application number: PCT/EP2013/051979
(87) International publication number: WO 2013/113857

(56) References cited:
- WO-A1-2005/066183
- US-A1- 2008 125 475
- US-A1- 2011 319 440

## Description

The invention relates to the treatment of fibromyalgia and chronic fatigue syndrome by administration of a pharmacologically active compound according to general formula (I) 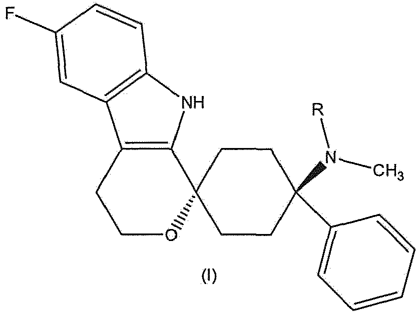 wherein R is -H or -CH₃, or a physiologically acceptable salt thereof. The treatment is effective for treating fibromyalgia-related pain and fatigue.

Fibromyalgia syndrome (FMS) is a chronic, widespread musculoskeletal pain and fatigue disorder, estimated to affect 2-4% of the population. FMS is characterized by a generalized heightened perception of sensory stimuli. Patients with FMS display abnormalities in pain perception in the form of both allodynia (pain with innocuous stimulation) and hyperalgesia (increased sensitivity to painful stimuli). The syndrome, as defined by the American College of Rheumatology's criteria, involves the presence of pain for over 3 months duration in all four quadrants of the body, as well as along the spine. In addition, pain is elicited at 11 out of 18 "tender points" upon palpation. In addition to muscle pain and fatigue, many patients commonly develop sleep and mood disorders (e.g., anxiety, depression). Patients also show a higher incidence of stress-related symptoms.

Chronic fatigue syndrome (CFS) is a debilitating disorder characterized by profound tiredness or fatigue. Patients with CFS may become exhausted with only light physical exertion, and must often function at a level of activity substantially lower than their capacity before the onset of illness. In addition to the key defining characteristic of fatigue, CFS patients generally report various nonspecific symptoms, including weakness, muscle aches and pains, excessive sleep, malaise, fever, sore throat, tender lymph nodes, impaired memory and/or mental concentration, insomnia, and depression. Like patients with FMS, patients with CFS suffer from disordered sleep, localized tenderness, and complaints of diffuse pain and fatigue.

Owing to their common symptomology, FMS and CFS are thought to be related. However, they manifest different major symptoms. Whereas pain is the major symptom reported by patients with FMS, fatigue is the major symptom reported by patients with CFS.

It is an object of the invention to provide pharmacologically active compounds that are useful in the treatment of fibromyalgia and chronic fatigue syndrome and that have advantages over the prior art.

This object has been achieved by the subject-matter of the patent claims.

The invention relates to a pharmaceutical dosage form comprising a pharmacologically active compound according to general formula (I) 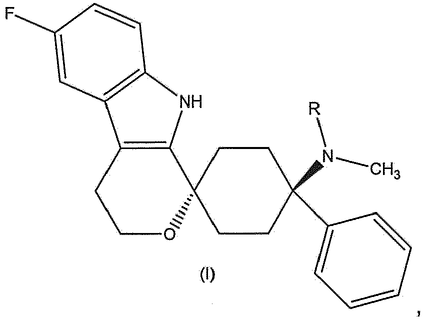 wherein R is -H or -CH₃,
or a physiologically acceptable salt thereof, for use in the treatment of fibromyalgia or chronic fatigue syndrome.
Figure 1 shows the effect of intravenous administration of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate's vehicle on spontaneous activity of *locus coeruleus* neurons.
Figure 2 shows the effect of intravenous administration of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate on spontaneous activity of *locus coeruleus* neurons.
Figure 3 shows a dose-effect curve illustrating the inhibitory effect of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate on *locus coeruleus* neuron firing rate.
Figure 4 shows withdrawal thresholds of the muscle before and after induction of muscle pain, and after treatment with (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate or vehicle.
Figure 5 shows dose response analysis for the effects of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate on muscle withdrawal thresholds when compared to vehicle.
Figure 6 shows withdrawal thresholds of the paw before and after induction of muscle pain, and after treatment with (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate or vehicle.
Figure 7 shows dose response analysis for the effects of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate on paw withdrawal thresholds when compared to vehicle.

For the purpose of the specification, fibromyalgia or chronic fatigue syndrome shall include conditions and symptoms that are associated with fibromyalgia or chronic fatigue syndrome, particularly pain due to fibromyalgia and pain due to chronic fatigue syndrome.

When the pharmaceutical dosage form is for the treatment of fibromyalgia or of a condition associated with fibromyalgia, the fibromyalgia is preferably selected from fibromyositis, fibrositis, myofibrositis, diffuse myofascial pain syndrome, primary fibromyalgia, secondary fibromyalgia, fibromyalgia-fibromyositis syndrome, fibromyositis-fibromyalgia syndrome, and muscular rheumatism.

When the pharmaceutical dosage form is for the treatment of chronic fatigue syndrome or of a condition associated with chronic fatigue syndrome, the chronic fatigue syndrome is preferably selected from chronic fatigue and immune dysfunction syndrome, chronic fatigue disorder, chronic fatigue-fibromyalgia syndrome, myalgic encephalomyelitis, postviral fatigue syndrome, chronic infectious mononucleosis-like syndrome, and royal free disease.

The pharmacologically active compound according to the invention is known from the prior art and can be administered orally, perorally, parenterally, intravenously, intraperitoneally, intradermally, intramuscularly, intrathecally, epidurally, intranasally, buccally, rectally or locally, for example to the skin, the mucous membranes or into the eyes. The compounds exhibit analgesic properties and are particularly suitable for the treatment of acute, visceral, neuropathic or chronic pain (cf., e.g., WO 2004/043967 and WO 2008/040481).

WO 2008/040481 discloses allodynia. However, allodynia is a clinical feature of many painful conditions, such as neuropathies, complex regional pain syndrome, postherpetic neuralgia, and migraine. Thus, the occurrence of allodynia does not indicate fibromyalgia or chronic fatigue syndrome. Rather, as fibromyalgia is a complex syndrome, diagnosis is not solely possible on the basis of the presence of allodynia. The key symptom of fibromyalgia patients is musculoskeletal pain. The diagnostic criteria proposed by the American College of Rheumatology include widespread pain in conjunction with tenderness on palpation of 11 or more of 18 specified tender points. For full diagnosis, further symptoms have to be taken into consideration, such as fatigue, mood disorders, and increased stress behavior.

Chronic musculoskeletal pain conditions remain somewhat refractory to treatment with currently available analgesics. Treatment of fibromyalgia syndrome and chronic fatigue syndrome needs a therapy which targets different symptoms, as the pharmacologically active compound of this invention.

Unless expressly stated otherwise, all dosages concerning the pharmacologically active compound according to the invention are preferably expressed as weight equivalent dosages based upon the free base.

The pharmacologically active compound of formula (I) is selected from 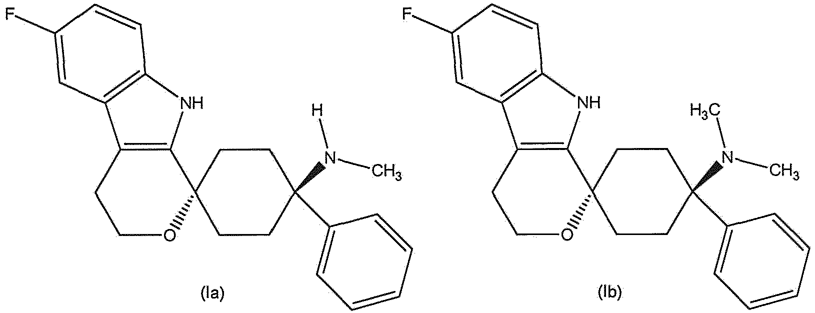 in the form of the free base or a physiologically acceptable salt thereof.

The free base according to general formula (Ia) can be systematically referred to as "1,1-(3-methylamino-3-phenylpentamethylene)-6-fluoro-1,3,4,9-tetrahydropyrano[3,4-b]indole (trans)" or as "(1r,4r)-6'-fluoro-N-methyl-4-phenyl-4',9'-dihydro-3'H-spiro[cyclohexane-1,1'-pyrano[3,4,b]indol]-4-amine", respectively.

The free base according to general formula (Ib) can be systematically referred to as "1,1-(3-dimethylamino-3-phenylpentamethylene)-6-fluoro-1,3,4,9-tetrahydropyrano[3,4-b]indole (trans)" or as "(1r,4r)-6'-fluoro-N,N-dimethyl-4-phenyl-4',9'-dihydro-3'H-spiro[cyclohexane-1,1'-pyrano[3,4,b]indol]-4-amine", respectively.

The definition of the pharmacologically active compound according to general formula (I) as used herein includes (1r,4r)-6'-fluoro-(N-methyl- or N,N-dimethyl-)-4-phenyl-4',9'-dihydro-3'H-spiro[cyclohexane-1,1'-pyrano[3,4,b]indol]-4-amine, derivatives thereof and stereoisomers thereof in any possible form, thereby particularly including solvates and polymorphs, salts, in particular acid addition salts and corresponding solvates and polymorphs.

The pharmacologically active compound according to general formula (I) may be present in form of the free base or in form of an acid addition salt, whereby any suitable acid capable of forming such an addition salt may be used.

The conversion of the pharmacologically active compound according to general formula (I) into a corresponding addition salt, for example, via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example, diethyl ether, diisopropyl ether, alkyl acetates, acetone and/or 2-butanone. Moreover, trimethylchlorosilane in aqueous solution is also suitable for the preparation of hydrochlorides.

The pharmacologically active compound according to general formula (I) is contained in the pharmaceutical dosage form in a therapeutically effective amount, i. e. in an amount that is therapeutically effective with regards to a daily administration of the pharmaceutical dosage form in the treatment of fibromyalgia or chronic fatigue syndrome. The amount that constitutes a therapeutically effective amount varies according to the compound, the condition being treated, the severity of said condition, the patient being treated, and whether the pharmaceutical dosage form is designed for an immediate or retarded release.

In a preferred embodiment, the pharmacologically active compound according to general formula (I) is contained in the pharmaceutical dosage form in a quantity such that single administration of the pharmaceutical dosage form does not lead to any analgesic effect, i.e. the pharmacologically active compound according to general formula (I) is contained in the pharmaceutical dosage form in an amount that is sub-therapeutic with regard to a single administration of the pharmaceutical dosage form. Preferably, however, once or twice daily administration of the pharmaceutical dosage form leads to an analgesic effect, at the latest, on the fifth day, more preferably at the latest on the fourth day and still more preferably at the latest on the third day of once daily administration.

In a preferred embodiment, the content of the pharmacologically active compound according to the general formula (I) in the pharmaceutical dosage form according to the invention is at most 95 wt.-%, more preferably at most 50 wt.-%, yet more preferably at most 25 wt.-%, still more preferably at most 10 wt.-%, even more preferably at most 5 wt.-%, most preferably at most 1.0 wt.-%, and in particular at most 0.5 wt.-%.

In another preferred embodiment, the content of the pharmacologically active compound according to the general formula (I) in the pharmaceutical dosage form according to the invention is at least 0.001 wt.-%, more preferably at least 0.005 wt.-%, yet more preferably at least 0.01 wt.-%, still more preferably at least 0.05 wt.-%, even more preferably at least 0.1 wt.-%, most preferably at least 0.5 wt.-%, and in particular at least 1.0 wt.-%.

Unless explicitly stated otherwise, in the meaning of the present invention the indication "wt.-%" shall mean weight of the respective ingredient per total weight of the pharmaceutical dosage form. In case that the pharmaceutical dosage form is film coated or encapsulated by an encapsulating medium which does not contain any amount of the pharmacologically active compound according to the general formula (I) and surrounds a core that in turn contains the total amount of the pharmacologically active compound according to the general formula (I), the indication "wt.-%" shall mean weight of the respective ingredient per total weight of the composition forming said core.

When the pharmaceutical dosage form is encapsulated or film coated, the pharmacologically active compound according to general formula (I) is preferably homogeneously distributed in the core of the pharmaceutical dosage form. Preferably, the encapsulating medium or film coating does not contain any pharmacologically active compound according to general formula (I).

Preferably, the pharmaceutical dosage form according to the invention is for administration once daily or twice daily.

The pharmaceutical dosage form according to the invention is preferably adapted for administration once daily and contains the pharmacologically active compound according to general formula (I) in a dose of preferably from 10 µg to 190 µg, or from 150 µg to 800 µg or 1,000 µg, or of more than 190 µg.

In a preferred embodiment, the dose of the pharmacologically active compound according to general formula (I) preferably is in the range of from 10 µg to 180 µg, preferably in the range of from 12.5 µg to 150 µg, more preferably in the range of from 15 µg to 120 µg, still more preferably in the range of from 17.5 µg to 100 µg, yet more preferably in the range of from 20 µg to 90 µg, most preferably in the range of from 25 µg to 80 µg, and in particular in the range of from 30 µg to 75 µg.

In another preferred embodiment, the dosage form according to the invention contains the pharmacologically active agent according to general formula (I) in a dose of from 150 µg to 800 µg, preferably more than 190 µg to 800 µg, more preferably more than 190 µg to 1,000 µg or 1,100 µg.

In a preferred embodiment, the dose of the pharmacologically active agent according to general formula (I) preferably is in the range of from 200 µg to 800 µg or 1,000 µg, preferably in the range of from 210 µg to 750 µg, more preferably in the range of from 220 µg to 700 µg, still more preferably in the range of from 230 µg to 650 µg, yet more preferably in the range of from 240 µg to 600 µg, and most preferably in the range of from 250 µg to 550 µg.

In a preferred embodiment, the dose of the pharmacologically active agent according to general formula (I) is in the range of from 200 µg to 600 µg. In a preferred embodiment, the dose of the pharmacologically active agent according to general formula (I) is in the range of from 300 µg to 500 µg.

In a preferred embodiment, the pharmaceutical dosage form according to the invention is adapted for oral administration. Suitable alternative pathways of administration of the pharmaceutical dosage form according to the invention include but are not limited to vaginal and rectal administration.

Preferably, the pharmaceutical dosage form according to the invention is intended for administration once daily.

For the purpose of the specification, "administration once daily" (sid, OD) preferably means that the pharmaceutical dosage form is adapted for being administered according to a regimen comprising the administration of a first pharmaceutical dosage form according to the invention and the subsequent administration of a second pharmaceutical dosage form according to the invention, wherein both, the first and the second pharmaceutical dosage form are administered during a time interval of about 48 hours, but wherein the second pharmaceutical dosage form is administered not earlier than 18 hours, preferably not earlier than 20 hours, more preferably not earlier than 22 hours and in particular, about 24 hours after the first pharmaceutical dosage form has been administered.

A skilled person is fully aware that administration regimens "once daily" may be realized by administering a single pharmaceutical dosage form containing the full amount of the pharmacologically active compound according to general formula (I) to be administered at a particular point in time or, alternatively, administering a multitude of dose units, i.e. two, three or more dose units, the sum of which multitude of dose units containing the full amount of the pharmacologically active compound according to general formula (I) to be administered at said particular point in time, where the individual dose units are adapted for simultaneous administration or administration within a short period of time, e.g. within 5, 10 or 15 minutes.

Preferably, the pharmaceutical dosage form according to the invention provides immediate release of the pharmacologically active compound according to general formula (I). The pharmaceutical dosage form is specifically designed to provide immediate release of the pharmacologically active compound according to general formula (I) *in vitro* in accordance with Ph. Eur. When the pharmaceutical dosage form is coated, e.g., with a coating that is soluble in gastric juice, the release kinetic is preferably monitored after such coating has been dissolved.

In a preferred embodiment, the pharmaceutical dosage form according to the invention is monolithic.

In another preferred embodiment, the pharmaceutical dosage form according to the invention comprises a core that is surrounded by a coating or by an encapsulating material. In a preferred embodiment, the core is liquid and the pharmacologically active compound according to general formula (I) is dispersed, preferably dissolved in the liquid.

Suitable additives and/or auxiliary substances to the compounds according to the invention in the process for the preparation of the pharmaceutical dosage form according to the invention are all the substances known to the expert from the prior art for achieving galenical formulations. The choice of these auxiliary substances and the amounts thereof to be employed depend on whether the pharmaceutical dosage form is to be administered orally, intravenously, intraperitoneally, intradermally, intramuscularly, intranasally, buccally or locally. Pharmaceutical dosage forms in the form of tablets, chewable tablets, coated tablets, capsules, granules, drops, juices or syrups are suitable for oral administration, and solutions, suspensions, easily reconstitutable dry formulations and sprays are suitable for parenteral, topical and inhalatory administration. Suppositories for use in the rectum are a further possibility.

Examples of auxiliary substances and additives for the oral administration forms are disintegrating agents, lubricants, binders, fillers, mould release agents, optionally solvents, flavourings, sugars, in particular carrier agents, diluents, dyestuffs, antioxidants etc. For suppositories, inter alia, waxes and fatty acid esters can be used, and for parenteral administration forms carrier substances, preservatives, suspension auxiliaries etc. can be used.

Auxiliary substances can be, for example: water, ethanol, 2-propanol, glycerol, ethylene glycol, propylene glycol, polyethylene glycol, polypropylene glycol, glucose, fructose, lactose, sucrose, dextrose, molasses, starch, modified starch, gelatine, sorbitol, inositol, mannitol, microcrystalline cellulose, methylcellulose, carboxymethylcellulose, cellulose acetate, shellac, cetyl alcohol, polyvinylpyrrolidone, paraffins, waxes, naturally occurring and synthetic gums, gum acacia, alginates, dextran, saturated and unsaturated fatty acids, stearic acid, magnesium stearate, zinc stearate, glyceryl stearate, sodium lauryl sulfate, edible oils, sesame oil, coconut oil, groundnut oil, soya bean oil, lecithin, sodium lactate, polyoxyethylene and -propylene fatty acid esters, sorbitan fatty acid esters, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulfate, zinc sulfate, calcium sulfate, potash, calcium phosphate, dicalcium phosphate, potassium bromide, potassium iodide, talc, kaolin, pectin, crospovidone, agar and bentonite.

The pharmaceutical formulations and pharmaceutical compositions are prepared with the aid of means, devices, methods and processes which are well-known in the prior art of pharmaceutical formulation, such as are described, for example, in "Remington's Pharmaceutical Sciences", ed. A. R. Gennaro, 17th ed., Mack Publishing Company, Easton, Pa. (1985), in particular in part 8, chapter 76 to 93.

Thus e.g. for a solid pharmaceutical dosage form, such as a tablet, the pharmacologically active compound or one of its physiologically acceptable salts, can be granulated with a pharmaceutical carrier, e.g. conventional tablet constituents, such as maize starch, lactose, sucrose, sorbitol, talc, magnesium stearate, dicalcium phosphate or pharmaceutically acceptable gums, and pharmaceutical diluents, such as e.g. water, in order to form a solid composition which comprises a compound according to the invention or a pharmaceutically acceptable salt thereof in homogeneous distribution. Homogeneous distribution is understood here as meaning that the active compound is uniformly distributed over the entire composition, so that this can easily be divided into unit dose forms, such as tablets, pills or capsules, having the same activity. The solid composition is then divided into unit dose forms. The tablets or pills of the pharmaceutical formulation according to the invention or of the compositions according to the invention can also be coated or compounded in another manner in order to provide a dose form with delayed release. Suitable coating compositions are, inter alia, polymeric acids and mixtures of polymeric acids with materials such as e.g. shellac, cetyl alcohol and/or cellulose acetate.

In a preferred embodiment, the pharmaceutical dosage form according to the invention provides the pharmacologically active compound according to general formula (I) in form of self-(micro) emulsifying drug delivery systems, solid solutions, nanoparticles, cyclodextrin complexes, liposomes, micelles, micronized and/or amorphous states.

In general terms, the options for formulation of poorly water-soluble drugs include crystalline solid, amorphous and lipid formulations.

The dissolution rate of the pharmacologically active compound from crystalline formulations can be increased by particle size reduction, thereby increasing the surface area for dissolution, e.g. by conventional micronization of the the pharmacologically active compound to particle sizes of about 2-5 µm. In some cases, this is not sufficient and nanocrystal technology is applied. Nanocrystals show a particle size of 100-250 nm, which can be obtained by ball-milling or by dense gas technology.

Solid solutions provide the pharmacologically active compound in an amorphous state immobilized in a polymer. Amorphous solutions may contain surfactants and polymers, thereby providing surface-activity during dispersion upon contact with water. Solid solutions can be formed using a variety of technologies such as spray drying and melt extrusion.

Lipid formulations exhibiting different characteristics can be used to disperse and form micellar solutions, including simple solutions and self-emulsifying drug delivery systems (SEDDS). Depending on the excipients, some require digestion (e. g. simple oily liquids), others can easily be absorbed without digestion.

Preferably, the pharmacologically active compound according to general formula (I) is molecularly dispersed in a matrix.

In a preferred embodiment, the pharmacologically active compound according to general formula (I) is molecularly dispersed in a non-crystalline matrix.

In another preferred embodiment, the pharmacologically active compound according to general formula (I) is molecularly dispersed in a non-amorphous matrix.

In a preferred embodiment, the pharmaceutical dosage form further contains a surfactant. Preferably, the surfactant is contained in a matrix in which the pharmacologically active compound according to general formula (I) is dispersed, preferably molecularly.

In a preferred embodiment, the pharmaceutical dosage form contains a surfactant. In another preferred embodiment, the pharmaceutical dosage form contains a mixture of two or more surfactants.

In a preferred embodiment, the surfactant acts as an O/W emulsifier. In another preferred embodiment, the surfactant acts as a W/O emulsifier.

Preferably, the pharmaceutical dosage form contains a surfactant having a hydrophilic-lipophilic balance (HLB) of at least 10 or at least 11. More preferably, the hydrophilic-lipophilic balance (HLB) is at least 12 or at least 13. Most preferably, the hydrophilic-lipophilic balance (HLB) ranges within 14 and 16.

Preferably, the hydrophilic-lipophilic balance (HLB) of the surfactant is at most 30, more preferably at most 28, still more preferably at most 26, yet more preferably at most 24, even more preferably at most 22, most preferably at most 20 and in particular at most 18.

In another preferred embodiment, the hydrophilic-lipophilic balance (HLB) of the surfactant is at least 27, more preferably at least 29, still more preferably at least 31, yet more preferably at least 33, even more preferably at least 35, most preferably at least 37 and in particular at least 39.

In a preferred embodiment, the HLB value of the surfactant is within the range of 10±3.5, more preferably 10±3, still more preferably 10±2.5, yet more preferably 10±2, even more preferably 10±1.5, most preferably 10±1, and in particular 10±0.5. In another preferred embodiment, the HLB value of the surfactant is within the range of 12±3.5, more preferably 12±3, still more preferably 12±2.5, yet more preferably 12±2, even more preferably 12±1.5, most preferably 12±1, and in particular 12±0.5. In still another preferred embodiment, the HLB value of the surfactant is within the range of 14±3.5, more preferably 14±3, still more preferably 14±2.5, yet more preferably 14±2, even more preferably 14±1.5, most preferably 14±1, and in particular 14±0.5. In another preferred embodiment, the HLB value of the surfactant is within the range of 15±3.5, more preferably 15±3, still more preferably 15±2.5, yet more preferably 15±2, even more preferably 15±1.5, most preferably 15±1, and in particular 15±0.5. In yet another preferred embodiment, the HLB value of the surfactant is within the range of 16±3.5, more preferably 16±3, still more preferably 16±2.5, yet more preferably 16±2, even more preferably 16±1.5, most preferably 16±1, and in particular 16±0.5. In another preferred embodiment, the HLB value of the surfactant is within the range of 18±3.5, more preferably 18±3, still more preferably 18±2.5, yet more preferably 18±2, even more preferably 18±1.5, most preferably 18±1, and in particular 18±0.5.

The surfactant can be ionic, amphoteric or non-ionic.

In a preferred embodiment, the pharmaceutical dosage form contains an ionic surfactant, in particular an anionic surfactant.

Suitable anionic surfactants include but are not limited to sulfuric acid esters such as sodium lauryl sulfate (sodium dodecyl sulfate, e.g. Texapon^{®} K12), sodium cetyl sulfate (e.g. Lanette E^{®}), sodium cetylstearyl sulfate, sodium stearyl sulfate, sodium dioctylsulfosuccinate (docusate sodium); and the corresponding potassium or calcium salts thereof.

Preferably, the anionic surfactant has the general formula (II-a)

CₙH₂ₙ₊₁O-SO₃⁻ M⁺ (II-a),

wherein n is an integer of from 8 to 30, preferably 10 to 24, more preferably 12 to 18;
and M is selected from Li⁺, Na⁺, K⁺, NH₄⁺ 1/2 Mg²⁺ and 1/2 Ca²⁺.

Further suitable anionic surfactants include salts of cholic acid including sodium glycocholate (e.g. Konakion^{®} MM, Cernevit^{®}), sodium taurocholate and the corresponding potassium or ammonium salts.

In another preferred embodiment, the pharmaceutical dosage form contains a non-ionic surfactant. Suitable non-ionic surfactants include but are not limited to
- fatty alcohols that may be linear or branched, such as cetylalcohol, stearylalcohol, cetylstearyl alcohol, 2-octyldodecane-1-ol and 2-hexyldecane-1-ol;
- sterols, such as cholesterole;
- partial fatty acid esters of sorbitan such as sorbitanmonolaurate, sorbitanmonopalmitate, sorbitanmonostearate, sorbitantristearate, sorbitanmonooleate, sorbitansesquioleate and sorbitantrioleate;
- partial fatty acid esters of polyoxyethylene sorbitan (polyoxyethylene-sorbitan-fatty acid esters), preferably a fatty acid monoester of polyoxyethylene sorbitan, a fatty acid diester of polyoxyethylene sorbitan, or a fatty acid triester of polyoxyethylene sorbitan; e.g. mono- and tri- lauryl, palmityl, stearyl and oleyl esters, such as the type known under the name "polysorbat" and commercially available under the trade name "Tween" including Tween^{®} 20 [polyoxyethylene(20)sorbitan monolaurate], Tween^{®} 21 [polyoxyethylene(4)sorbitan monolaurate], Tween^{®} 40 [polyoxyethylene(20)sorbitan monopalmitate], Tween^{®} 60 [polyoxyethylene(20)sorbitan monostearate], Tween^{®} 65 [polyoxyethylene(20)sorbitan tristearate], Tween^{®} 80 [polyoxyethylene(20)sorbitan monooleate], Tween 81 [polyoxyethylene(5)sorbitan monooleate], and Tween^{®} 85 [polyoxyethylene(20)sorbitan trioleate]; preferably a fatty acid monoester of polyoxyethylenesorbitan according to general formula (II-b) 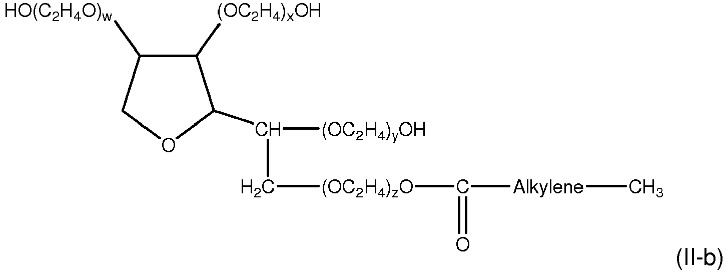
   wherein (w+x+y+z) is within the range of from 15 to 100, preferably 16 to 80, more preferably 17 to 60, still more preferably 18 to 40 and most preferably 19 to 21;
   and alkylene is an optionally unsaturated alkylene group comprising 6 to 30 carbon atoms, more preferably 8 to 24 carbon atoms and most preferably 10 to 16 carbon atoms;
- polyoxyethyleneglycerole fatty acid esters such as mixtures of mono-, di- and triesters of glycerol and di- and monoesters of macrogols having molecular weights within the range of from 200 to 4000 g/mol, e.g., macrogolglycerolcaprylocaprate, macrogolglycerollaurate, macrogolglycerolococoate, macrogolglycerollinoleate, macrogol-20-glycerolmonostearate, macrogol-6-glycerolcaprylocaprate, macrogolglycerololeate; macrogolglycerolstearate, macrogolglycerolhydroxystearate (e.g. Cremophor^{®} RH 40), and macrogolglycerolrizinoleate (e.g. Cremophor^{®} EL);
- polyoxyethylene fatty acid esters, the fatty acid preferably having from about 8 to about 18 carbon atoms, e.g. macrogololeate, macrogolstearate, macrogol-15-hydroxystearate, polyoxyethylene esters of 12-hydroxystearic acid, such as the type known and commercially available under the trade name "Solutol HS 15"; preferably according to general formula (II-c)

   CH₃CH₂-(OCH₂CH₃)ₙ-O-CO-(CH₂)ₘCH₃ (II-C)

   wherein n is an integer of from 6 to 500, preferably 7 to 250, more preferably 8 to 100, still more preferably 9 to 75, yet more preferably 10 to 50, even more preferably 11 to 30, most preferably 12 to 25, and in particular 13 to 20; and
   wherein m is an integer of from 6 to 28; more preferably 6 to 26, still more preferably 8 to 24, yet more preferably 10 to 22, even more preferably 12 to 20, most preferably 14 to 18 and in particular 16;
- polyoxyethylene fatty alcohol ethers, e.g. macrogolcetylstearylether, macrogollarylether, macrogololeylether, macrogolstearylether;
- polyoxypropylene-polyoxyethylene block copolymers (poloxamers);
- fatty acid esters of saccharose; e.g. saccharose distearate, saccharose dioleate, saccharose dipalmitate, saccharose monostearate, saccharose monooleate, saccharose monopalmitate, saccharose monomyristate and saccharose monolaurate;
- fatty acid esters of polyglycerol, e.g. polyglycerololeate;
- polyoxyethylene esters of alpha-tocopheryl succinate, e.g. D-alpha-tocopheryl-PEG-1000-succinate (TPGS);
- polyglycolyzed glycerides, such as the types known and commercially available under the trade names "Gelucire 44/14", "Gelucire 50/13 and "Labrasol";
- reaction products of a natural or hydrogenated castor oil and ethylene oxide such as the various liquid surfactants known and commercially available under the trade name "Cremophor"; and
- partial fatty acid esters of multifunctional alcohols, such as glycerol fatty acid esters, e.g. mono- and tri-lauryl, palmityl, stearyl and oleyl esters, for example glycerol monostearate, glycerol monooleate, e.g. glyceryl monooleate 40, known and commercially available under the trade name "Peceol"; glycerole dibehenate, glycerole distearate, glycerole monolinoleate; ethyleneglycol monostearate, ethyleneglycol monopalmitostearate, pentaerythritol monostearate.

In a preferred embodiment, the content of the surfactant is at least 0.001 wt.-% or at least 0.005 wt.-%, more preferably at least 0.01 wt.-% or at least 0.05 wt.-%, still more preferably at least 0.1 wt.-%, at least 0.2 wt.-%, or at least 0.3 wt.-%, yet more preferably at least 0.4 wt.-%, at least 0.5 wt.-%, or at least 0.6 wt.-%, and in particular at least 0.7 wt.-%, at least 0.8 wt.-%, at least 0.9 wt.-%, or at least 1.0 wt.-%, based on the total weight of the pharmaceutical dosage form.

In another preferred embodiment, particularly when the pharmaceutical dosage form contains an encapsulated core, the content of the surfactant is at least 10 wt.-%, more preferably at least 15 wt.-%, still more preferably at least 20 wt.-%, yet more preferably at least 25 wt.-% and in particular at least 30 wt.-%, based on the total weight of the composition forming the core. In a preferred embodiment, the content of the surfactant ranges preferably from 0.1 wt.-% to 95 wt.-%, more preferably from 1 wt.-% to 95 wt.-%, still more preferably from 5 wt.-% to 90 wt.-%, yet more preferably from 10 wt.-% to 80 wt.-%, most preferably from 20 wt.-% to 70 wt.-%, and in particular from 30 wt.-% to 75 wt.-%, based on the total weight of the composition forming the core.

In a particular preferred embodiment,
- the pharmaceutical dosage form is for administration to a mammal, preferably to a human, more preferably to a female; and/or
- the pharmaceutical dosage form is for administration to an adult (i.e. a human of the age of 18 years or more), preferably a geriatric patient (i.e. a human of the age of at least 65, or at least 70, or at least 75 or at least 80 years); and/or
- the fibromyalgia is selected from fibromyositis, fibrositis, myofibrositis, diffuse myofascial pain syndrome, primary fibromyalgia, secondary fibromyalgia, fibromyalgia-fibromyositis syndrome, fibromyositis-fibromyalgia syndrome, and muscular rheumatism.

In another preferred embodiment,
- the pharmaceutical dosage form contains the pharmacologically active compound in an amount of more than 190 µg and/or
- the pharmaceutical dosage form is for administration twice daily.

A further aspect of the invention relates to a method of treating fibromyalgia or chronic fatigue syndrome comprising the administration of a pharmacologically effective amount of the pharmacologically active compound according to general formula (I) or a physiologically acceptable salt thereof; preferably oral administration of the pharmaceutical dosage form according to the invention; to a subject in need thereof.

The following examples further illustrate the invention but are not to be construed as limiting its scope.

### EXAMPLE 1:

Patients suffering from fibromyalgia report an increased incidence of stress. It is well established that acute stress exposure can increase the discharge activity and norepinephrine release from noradrenergic *locus coeruleus* (LC) neurons. The LC contains the largest aggregate of noradrenergic neurons in the mammalian brain. Chronic exposure to stress can alter the response of LC neurons to subsequent stress exposure. It is to be expected that a compound which is able to inhibit the discharge activity within the LC would be appropriate for treating stress-related symptoms.

(1r,4r)-6'-fluoro-N,N-dimethyl-4-phenyl-4',9'-dihydro-3'H-spiro[cyclohexane-1,1'-pyrano-[3,4,b]indol]-4-amine hemi-citrate elicited an inhibitory effect on the spontaneous activity of LC neurons in the rat after acute treatment. The inhibition was complete and the ED₅₀ value estimated from the dose-effect curves was 8.08 ± 0.87 µg/kg i.v. (cumulative dose).

### Method:

The experiments were all carried out in male albino Sprague-Dawley rats weighing 265-313 g. Animals were maintained under standard laboratory conditions (21 °C, 12h light/dark cycle, lights on at 8:00 AM, food and water ad libitum). Every effort was made to minimize animal suffering and to use the minimum possible number of animals. Animal use procedures were conformed to European Ethical Standards (86/609-EEC) and Spanish law (RD1201/2005) for the care and use of laboratory animals. The experimental protocols were reviewed and approved by the Committee for Animal Experimentation at the University of Cádiz and complied with the International Association for the Study of Pain Ethical Guidelines.

Rats were anesthetized with chloral hydrate (400 mg/kg i.p.); subsequently, a cannula was inserted into the trachea and the right jugular vein was cannulated for systemic i.v. injections of anaesthetic and drugs. Supplemental doses of anaesthetic were given to prevent any nociceptive reaction to pinching of the hind paw. Body temperature was maintained at 37 °C with a heating pad. The rat was placed in a stereotaxic frame with its head at a 15° angle to the horizontal plane (nose down). To approach the *locus coeruleus,* the skull was exposed, and a hole (approximately 3 mm diameter) was drilled for the insertion of the recording electrode at 1.1 mm lateral to the midline and 3.7 mm posterior to the lamboid fontanel over the cerebellum. The dura over the cerebellum was carefully removed.

To study the acute effect of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate on *locus coeruleus* neurons *in vivo,* dose-effect curves were performed for (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate. It was injected at 2 min intervals, in increasing doses, until maximal effect was reached. Furthermore, the possible role of the vehicle used to dissolve (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate on the activity of *locus coeruleus* neurons was studied. For this purpose, the same protocol used to study the effect of (1r,4r)-6'-fluoro-N,N-dimethyl-4-phenyl-4' ,9'-dihydro-3'H-spiro[cyclohexane-1,1'-pyrano-[3,4,b]indol]-4-amine hemi-citrate was followed.

### Electrophysiological recording of locus coeruleus neurons in vivo:

The recording electrode was an Omegadot single-barrel glass micropipette filled with a 2% solution of Pontamine Sky Blue in 0.5% sodium acetate and broken back to a tip diameter of 1 - 2.5 µm. The extracellular signal from the electrode was amplified, discriminated and monitored on an oscilloscope and with an audio monitor too. Discriminated spikes were fed into a PC and processed using computer software (CED micro 1401 interface and Spike2 software, Cambridge Electronic Design, U.K.). *Locus coeruleus* neurons were encountered 5.5 - 6.0 mm below the dural surface, just ventral to a zone of relative silence (corresponding to the IV^{th} ventricle), and medial to neurons of the mesencephalic nucleus of the V^{th} cranial nerve (which could be activated by depression of the mandible). *Locus coeruleus* neurons were identified by standard criteria that included: long duration action potential (> 2 ms); spontaneous firing at a regular rhythm; a slow firing rate; and characteristic spikes with a long-lasting positive-negative waveform. The basal firing rate was recorded at least 2 min prior to any drug administration. Only one noradrenergic *locus coeruleus* cell was pharmacologically studied in each animal.

**Table 1: Scheme of administration of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate:**

| **Administration volume (ml/kg)** | **Solution** | **Cumulative dose (µg/kg)*** |
|---|---|---|
| 0.25 | Solution 1 (8.75 (µg/kg) | 2.1875 |
| 0.25 | | 4.3750 |
| 0.5 | | 8.7500 |
| 0.25 | Solution 2 (35.00 µg/kg) | 17.5000 |
| 0.25 | | 26.2500 |
| 0.25 | | 35.0000 |

| | | |
|---|---|---|
| * doses have been expressed with one decimal number in Figure 2 and Table 2. | | |

This scheme of administration was also followed to study the effect of the vehicle for (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate.

### Data and statistical analysis:

Frequency rate was expressed in hertz (Hz) and the changes in firing rate as percentages of the baseline firing rate (defined as 0%). Dose-concentration-effect curves were analyzed for the best non-linear fit to a logistic three-parameter equation: E = Eₘₐₓ [A]ⁿ / (end₅₀ⁿ + [A]ⁿ), where [A] is the i.v. dose of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate and E is the effect on the firing rate induced by A; Eₘₐₓ is the maximal percentage change at "infinite" dose (100 %); ED₅₀ is the effective dose for eliciting 50 % of Eₘₐₓ; n is the slope factor of the dose-response curve. Experimental data were analyzed by using the computer program Graphd Prism (v. 3.0; GraphPad Software, Inc.). Statistical significance was assessed by means of a one-way repeated measures analysis of variance (ANOVA) to study the effect of compounds on spontaneous firing rate. To analyze the firing rate before and after drug administration, unpaired Student's t-tests was used. The level of significance was considered as p < 0.05. Data are reported as mean ± S.E.M.

### Results:

### a) Effect of acute administration of the vehicle on the activity of locus coeruleus neurons (Step 1):

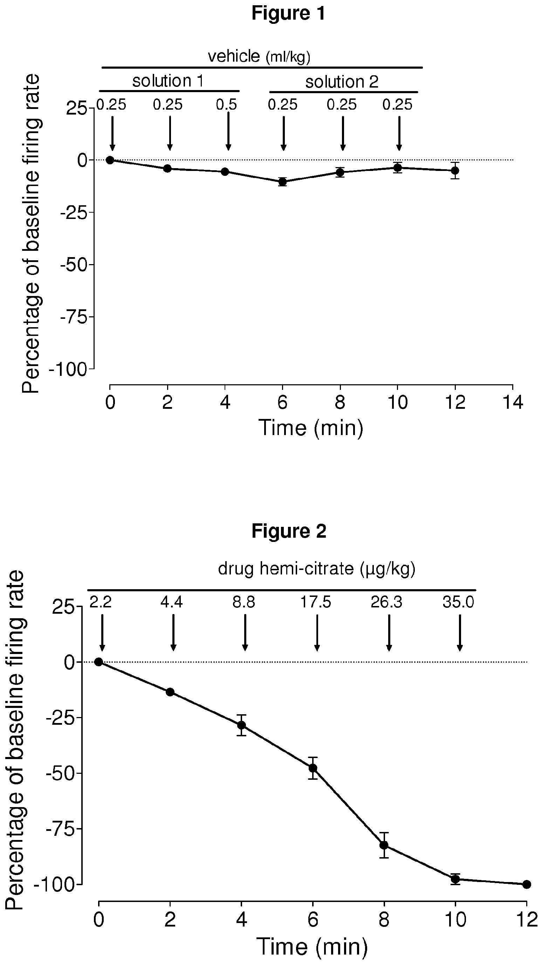

Vehicle administration did not modify the spontaneous activity of *locus coeruleus* neurons (F_{(4,29)} = 1.24, p > 0.05, one-way repeated measures ANOVA; n = 5; Figure 1).

Figure 1 shows the effect of intravenous administration of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate's vehicle on spontaneous activity of *locus coeruleus* neurons. Symbols represent mean ± S.E.M. Note that (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate's vehicle did not modify the spontaneous activity. The horizontal dashed line represents baseline unit activity.

### b) Effect of acute administration of (1r,4r)-6'-fluoro-N,N-dimethyl-4-phenyl-4',9'-dihydro-3'H-spiro[cyclohexane-1,1-pyrano-[3,4,b]indol]-4-amine hemi-citrate on the firing activity of locus coeruleus neurons (Step 2):

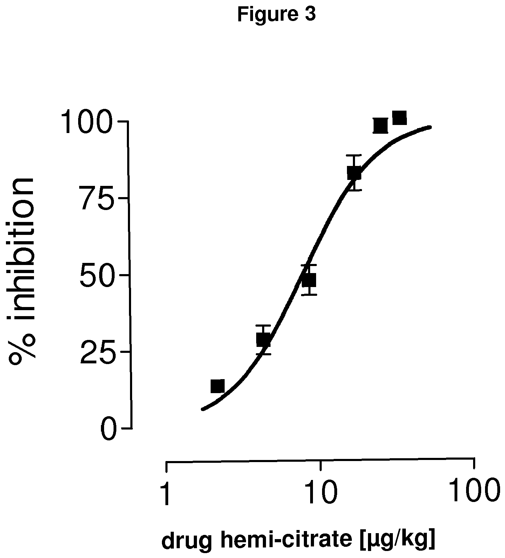

Administration of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate depressed the spontaneous activity of *locus coeruleus* neurons in a dose-dependent manner (F_{(4,29)} = 3.75, p < 0.0001, one-way repeated measures ANOVA; n = 5; Figure 2, Figure 3, Table 2). Complete inhibition was achieved in all cells tested and the mean ED₅₀ value estimated from the dose-effect curves was 8.08 ± 0.87 µg/kg i.v. (cumulative dose; n = 5).

Figure 2 shows the effect of intravenous administration of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate on spontaneous activity of *locus coeruleus* neurons. Symbols represent mean ± S.E.M.. The horizontal dashed line represents baseline unit activity.

**Table 2: Effect of cumulative doses of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate (="drug") on spontaneous activity of locus coeruleus neurons. Data are expressed as mean ± S.E.M. frequency (Hz) and as percentage of reduction from basal firing rate:**

| **Dose drug (µg/kg)** | **Frequency (Hz)** | **% of baseline firing rate** |
|---|---|---|
| 0 | 1.76±0.17 | |
| 2.2 | 1.39±0.10 | -13.48±1.82 |
| 4.4 | 1.25±0.13 | -28.36±4.62 |
| 8.8 | 0.92±0.14 | -49.19±4.91 |
| 17.5 | 0.33±0.14 | -82.33±5.70 |
| 26.3 | 0.06±0.06 | -97.64±2.36 |
| 35.0 | 0.00±0.00 | -100.00±0.00 |

Figure 3 shows a dose-effect curve illustrating the inhibitory effect of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate on *locus coeruleus* neuron firing rate. Symbols represent mean ± S.E.M. of the percentage of reduction from basal firing rate. The horizontal axis represents the cumulative doses of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate administered i.v. at 2 min intervals.

### Conclusion:

The above experimental results indicate that (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate elicited an inhibitory effect on the spontaneous activity of *locus coeruleus* neurons in acute treatment. The inhibition reached by (1r,4r)-6'-fluoro-N,N-dimethyl-4-phenyl-4',9'-dihydro-3'H-spiro-[cyclohexane-1,1'-pyrano-[3,4,b]indol]-4-amine hemi-citrate was complete and the ED₅₀ value estimated from the dose-effect curves was 8.08 ± 0.87 µg/kg i.v. (cumulative dose).

### EXAMPLE 2:

Musculoskeletal pain is the hallmark of fibromyalgia. A model has been developed by K. A. Sluka which has greater face validity to pain of musculoskeletal origin in humans.

This model is characterized by robust muscle (primary) and cutaneous (secondary) hyperalgesia induced by repeated intramuscular acid injections.

### Method:

### Experimental procedures:

The experiments were performed on adult Sprague-Dawley rats weighing 250-350 g housed in transparent plastic cages with free access to food and water, in a 12 h light-dark cycle. All the experimental procedures were approved by the Animal Care and Use Committee at the University of Iowa.

### Non-Inflammatory Muscle Pain:

All rats were anesthetized with 2%-3% isoflurane. The left gastrocnemius muscle was injected with 100 µL of pH 4.0 saline, 5 days apart. 24 hours after the second injection of acidic saline mechanical withdrawal thresholds of the paw and muscle were measured.

### Behavior test: mechanical withdrawal threshold of the muscle:

Rats were acclimated to the room for 20 minutes. Rats were acclimated for 2 days, 2 times per day for 5 minutes to a gardener's glove prior to testing. To test for muscle withdrawal thresholds the rat was placed in a gardener's glove and the gastrocnemius was squeezed with a tweezer apparatus until a withdrawal of the hindlimb. This was repeated three times and averaged to obtain a muscle withdrawal threshold.

### Behavior test: Mechanical withdrawal threshold of the paw:

Rats were acclimated to the room for 20 minutes and to the testing transparent plastic cages on elevated wire mesh floor for 15 minutes for 2 days prior to testing. To test for mechanical withdrawal thresholds of the paw calibrated von Frey filaments with bending forces ranging from 1 to 210 mN were applied to the both the ipsilateral and contralateral paws. Each filament was applied for approximately 1 s with enough to bend the filaments. Each filament was applied twice and a positive response was one withdrawal. Once a positive response was found, the filament above and below the filament that caused a positive response was tested. Confirmation of withdrawal threshold was established if there was a positive withdrawal from the filament above and no withdrawal from the filament below. The lowest withdrawal force that produced a withdrawal was recorded as the threshold. A decrease in mechanical withdrawal threshold of the paw is interpreted as cutaneous hyperalgesia of the paw.

### Effects of (1r,4r)-6'-fluoro-N,N-dimethyl-4-phenyl-4',9'-dihydro-3'H-spiro[cyclohexane-1,1-pyrano-[3,4,b]indol]-4-amine hemi-citrate on pain behaviors in animal model of non-inflammatory muscle pain:

The effects of four doses of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate (1, 3, 10, 30 µg/kg) and vehicle (10% DMSO/ 5% Cremophor EL/ 85% Glucose solution 5%) on the non-inflammatory model for muscle pain were tested. 24 hours after induction of the model paw and muscle withdrawal thresholds were measured. Once a decrease in withdrawal thresholds was established the rats were injected with one of the doses of the compound (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate or the vehicle intraperitoneally. Rats were then tested for withdrawal thresholds 15 minutes, 30 minutes, 1 hour, or 2 hours after drug. Blood was removed by direct puncture of the heart after experiment and collected in Lithium-Heparin tubes. Blood was centrifuged at 2375 g for 10 minutes at 4°C and the plasma was collected and stored at -20°C.

### Statistical analysis:

Groups of 8 rats were used for each dose except for the 3 µg/kg dose of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate which had 7 rats.

A repeated measures ANOVA compared differences between withdrawal thresholds time across time and between groups. Post-hoc testing compared differences between groups with a Tukey's test. The overall change in withdrawal thresholds of the paw or muscle were calculated as an area under the curve (ipsilaterally) to create a dose-response analysis. A one-way ANOVA followed by post-hoc Tukey's test examined for differences between groups at individual time periods and for area. P<0.05 was considered statistically significant. Data are presented as the means with the SEM.

### Results:

### Muscle withdrawal thresholds:

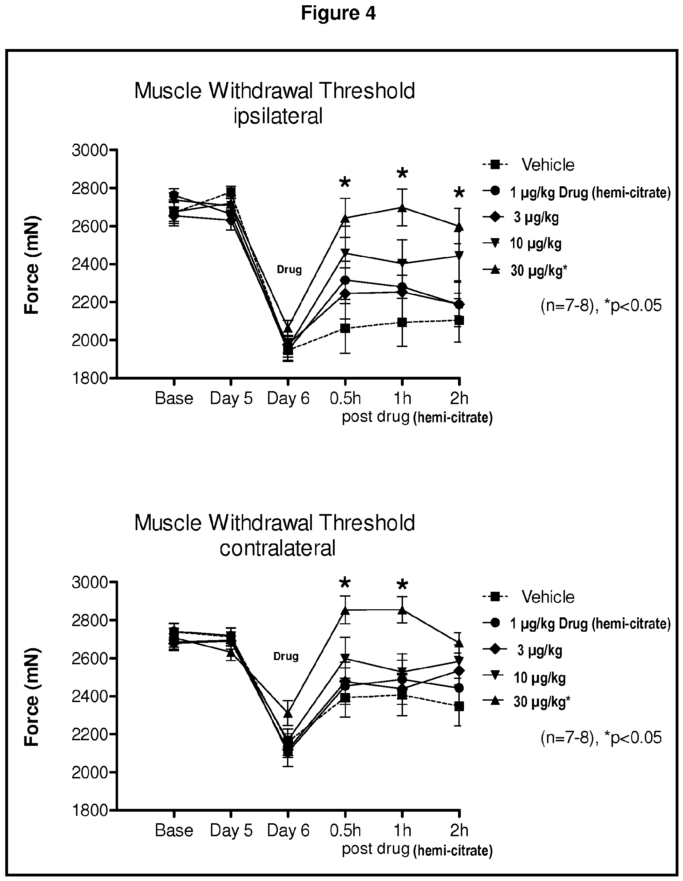

24h after the second injection of acidic saline, withdrawal thresholds of the paw were significantly decreased in all groups, bilaterally (Figure 4). There was a significant difference between groups after treatment with drug or vehicle both ipsilaterally and contralaterally. The highest dose of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate was significantly greater than vehicle 30 min, 1 h and 2h after delivery ipsilaterally, and 30 min and 1 h after delivery contralaterally.

Figure 4 shows withdrawal thresholds of the muscle before and after induction of muscle pain, and after treatment with (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate or vehicle. Each point of the graph represents the mean ± standard error of the mean (SEM); number of animals per group = 7-8 (* p<0.05).

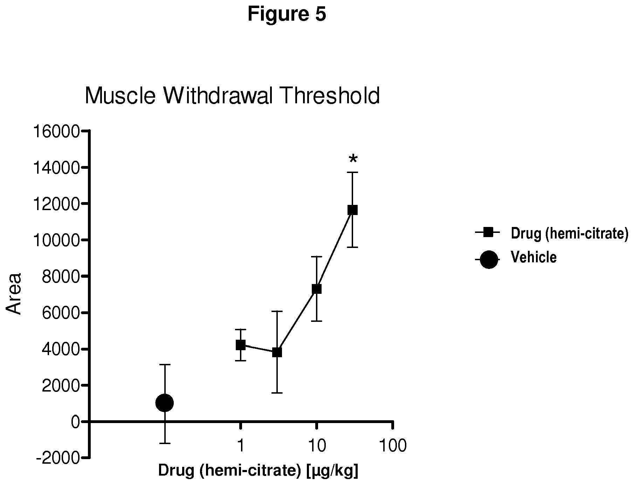

When analyzing dose-response relationships the 30 µg/kg (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate group increased withdrawal thresholds of the muscle when compared to vehicle, 1 µg/kg and 3 µg/kg (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate (Figure 5).

Figure 5 shows dose response analysis for the effects of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate when compared to vehicle. The area under the curve for the 2 hour testing period was calculated for the analysis.

### Paw withdrawal thresholds:

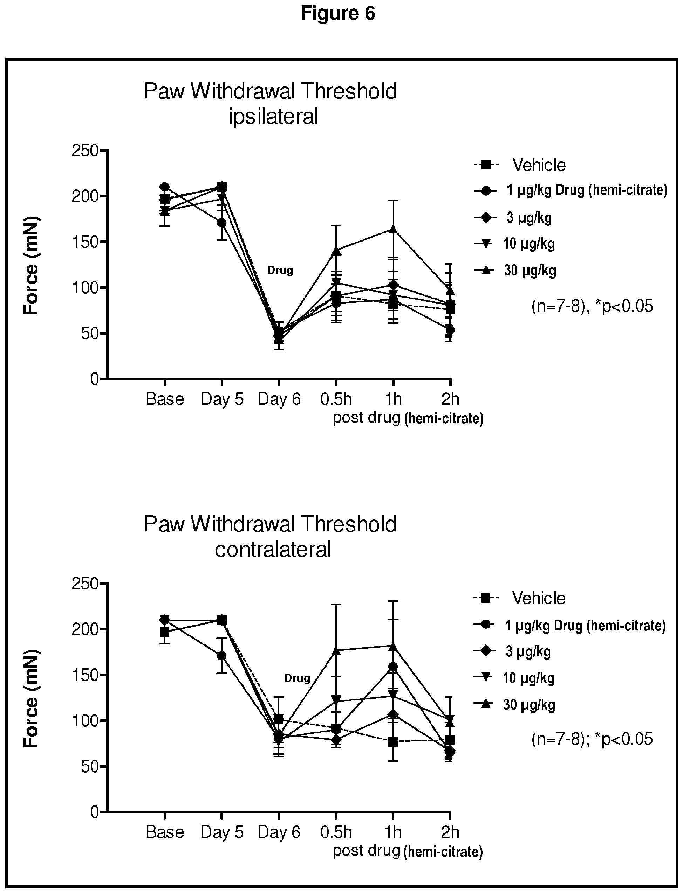

24h after the second injection of acidic saline, withdrawal thresholds of the paw were significantly decreased in all groups, bilaterally. However, (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate did not significantly increase the withdrawal thresholds of the paw when compared to vehicle (Figure 6). There was a trend towards an increase in withdrawal thresholds of the paw after the highest dose of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate. There were increases in 5/8 animals with the highest dose of (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate showing increases in withdrawal thresholds ipsilaterally to within normal uninjured range.

Figure 6 shows withdrawal thresholds of the paw before and after induction of muscle pain, and after treatment with (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate or vehicle. Each point of the graph represents the mean ± standard error of the mean (SEM); number of animals per group = 7-8 (* p<0.05).

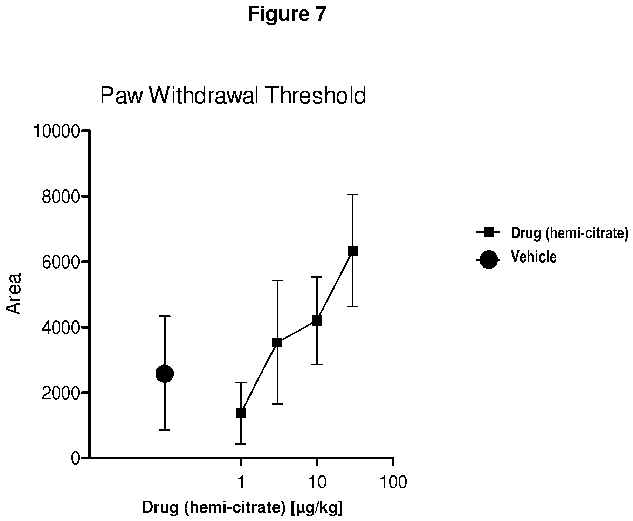

Dose-response analysis similarly showed non-significant changes in withdrawal thresholds with (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate compared to vehicle controls (Figure 7).

Figure 7 shows dose response analysis for the effects of (1 r,4r)-6'-fluoro-N,N-dimethyl-4-phenyl-4',9'-dihydro-3'H-spiro[cyclohexane-1,1'-pyrano-[3,4,b]indol]-4-amine hemi-citrate when compared to vehicle. The area under the curve for the 2 hour testing period was calculated for the analysis. Each point of the graph represents the mean ± standard error of the mean (SEM); number of animals per group = 7-8 (* p<0.05).

### Conclusion:

These results show that compound (1r,4r)-6'-fluoro-N,N-dimethyl-4-phenyl-4',9'-dihydro-3'H-spiro[cyclohexane-1,1'-pyrano-[3,4,b]indol]-4-amine hemi-citrate dose-dependently decreases muscle hyperalgesia in a model of chronic muscle pain.

(1r,4r)-6'-fluoro-N,N-dimethyl-4-phenyl-4',9'-dihydro-3'H-spiro[cyclohexane-1,1'-pyrano-[3,4,b]indol]-4-amine hemi-citrate shows a trend towards an effect on secondary cutaneous hyperalgesia induced by chronic muscle pain.

## Claims

1. A pharmaceutical dosage form comprising a pharmacologically active compound according to general formula (I) 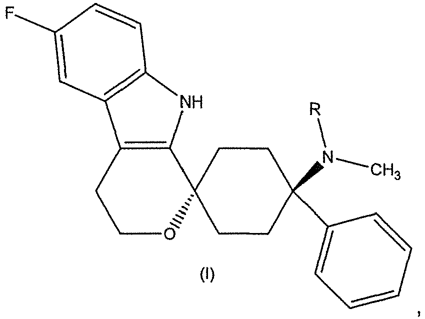 wherein R is -H or -CH₃,
or a physiologically acceptable salt thereof,
for use in the treatment of fibromyalgia or chronic fatigue syndrome.

2. The pharmaceutical dosage form according to claim 1 for use in the treatment of fibromyalgia or chronic fatigue syndrome according to claim 1, wherein the pharmacologically active compound is (1r,4r)-6'-fluoro-N,N-dimethyl-4-phenyl-4',9'-dihydro-3'H-spiro[cyclo¬hexane-1,1'-pyrano¬[3,4,b]indol]-4-amine or a physiologically acceptable salt thereof.

3. The pharmaceutical dosage form according to claim 1 or 2 for use in the treatment of fibromyalgia or chronic fatigue syndrome according to claim 1 or 2, wherein the fibromyalgia is selected from fibromyositis, fibrositis, myofibrositis, diffuse myofascial pain syndrome, primary fibromyalgia, secondary fibromyalgia, fibromyalgia-fibromyositis syndrome, fibromyositis-fibromyalgia syndrome, and muscular rheumatism.

4. The pharmaceutical dosage form according to any of the preceding claims for use in the treatment of fibromyalgia or chronic fatigue syndrome according to any of the preceding claims, which is for administration once daily or twice daily.

5. The pharmaceutical dosage form according to any of the preceding claims for use in the treatment of fibromyalgia or chronic fatigue syndrome according to any of the preceding claims, which is for oral administration.

6. The pharmaceutical dosage form according to claim 5 for use in the treatment of fibromyalgia or chronic fatigue syndrome according to claim 5, which is selected from the group consisting of tablets, chewable tablets, coated tablets, capsules, granules, drops, juices or syrups.

7. The pharmaceutical dosage form according to any of the preceding claims for use in the treatment of fibromyalgia or chronic fatigue syndrome according to any of the preceding claims, which is for administration to a mammal, preferably to a human.

8. The pharmaceutical dosage form according to any of the preceding claims for use in the treatment of fibromyalgia or chronic fatigue syndrome according to any of the preceding claims, which is for administration to an adult.

9. The pharmaceutical dosage form according to claim 8 for use in the treatment of fibromyalgia or chronic fatigue syndrome according to claim 8, wherein the adult is a geriatric patient.

## Patentansprüche

1. Pharmazeutische Darreichungsform umfassend eine pharmakologisch aktive Verbindung gemäß der allgemeinen Formel (I) 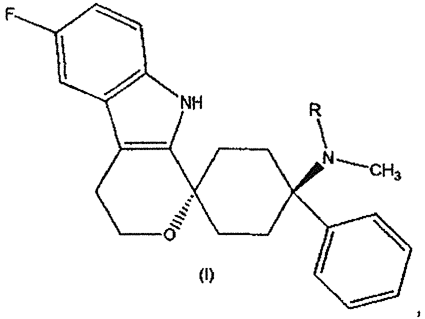 wobei R -H oder -CH₃ ist,
oder ein physiologisch annehmbares Salz davon ist,
zur Verwendung in der Behandlung von Fibromyalgie oder chronischem Ermüdungssyndrom.

2. Pharmazeutische Darreichungsform nach Anspruch 1 zur Verwendung in der Behandlung von Fibromyalgie oder chronischem Ermüdungssyndrom nach Anspruch 1, wobei die pharmakologisch aktive Verbindung (1r,4r)-6'-Fluor-N,N-dimethyl-4-phenyl-4',9'-dihydro-3'H-spiro[cyclohexan-1,1'-pyrano¬[3,4,b]indol]-4-amin oder ein physiologisch annehmbares Salz davon ist.

3. Pharmazeutische Darreichungsform nach Anspruch 1 oder 2 zur Verwendung in der Behandlung von Fibromyalgie oder chronischem Ermüdungssyndrom nach Anspruch 1 oder 2, wobei die die Fibromyalgie ausgewählt ist von Fibromyositis, Fibrositis, Myofibrositis, diffusem myofaszialem Schmerzsyndrom, primärer Fibromyalgie, sekundärer Fibromyalgie, Fibromyalgie-Fibromyositis-Syndrom, Fibromyositis-Fibromyalgie-Syndrom und Muskelrheumatismus.

4. Pharmazeutische Darreichungsform nach einem der vorherigen Ansprüche zur Verwendung in der Behandlung von Fibromyalgie oder chronischem Ermüdungssyndrom nach einem der vorherigen Ansprüche, die zur Verabreichung einmal täglich oder zweimal täglich ist.

5. Pharmazeutische Darreichungsform nach einem der vorherigen Ansprüche zur Verwendung in der Behandlung von Fibromyalgie oder chronisches Ermüdungssyndrom nach einem der vorherigen Ansprüche, die zur oralen Verabreichung ist.

6. Pharmazeutische Darreichungsform nach Anspruch 5 zur Verwendung in der Behandlung von Fibromyalgie oder chronischem Ermüdungssyndrom nach Anspruch 5, die ausgewählt ist von der Gruppe bestehend aus Tabletten, kaubaren Tabletten, beschichteten Tabletten, Kapseln, Körnern, Tropfen, Säften oder Sirups.

7. Pharmazeutische Darreichungsform nach einem der vorherigen Ansprüche zur Verwendung in der Behandlung von Fibromyalgie oder chronisches Ermüdungssyndrom nach einem der vorherigen Ansprüche, die zur Verabreichung an ein Säugetier, vorzugsweise einen Menschen ist.

8. Pharmazeutische Darreichungsform nach einem der vorherigen Ansprüche zur Verwendung in der Behandlung von Fibromyalgie oder chronisches Ermüdungssyndrom nach einem der vorherigen Ansprüche, die zur Verabreichung an einen Erwachsenen ist.

9. Pharmazeutische Darreichungsform nach Anspruch 8 zur Verwendung in der Behandlung von Fibromyalgie oder chronischem Ermüdungssyndrom nach Anspruch 8, wobei der Erwachsene ein Geriatrie-Patient ist.

## Revendications

1. Forme posologique pharmaceutique comprenant un composé pharmacologiquement actif de formule générale (I) 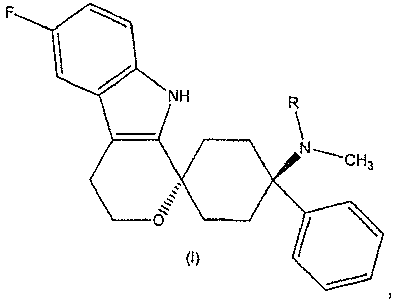 dans laquelle R représente -H ou un groupe -CH₃,
ou un de ses sels physiologiquement acceptables,
pour une utilisation dans le traitement de la fibromyalgie ou du syndrome de fatigue chronique.

2. Forme posologique pharmaceutique suivant la revendication 1, pour une utilisation dans le traitement de la fibromyalgie ou du syndrome de fatigue chronique suivant la revendication 1, dans laquelle le composé pharmacologiquement actif est la (1r,4r)-6'-fluoro-N,N-diméthyl-4-phényl-4',9'-dihydro-3'H-spiro[cyclohexane-1,1'-pyrano[3,4,b]indole]-4-amine ou un de ses sels physiologiquement acceptables.

3. Forme posologique pharmaceutique suivant la revendication 1 ou 2, pour une utilisation dans le traitement de la fibromyalgie ou du syndrome de fatigue chronique suivant la revendication 1 ou 2, la fibromyalgie étant choisie entre la fibromyosite, la fibrosite, la myofibrosite, le syndrome de douleur myofaciale diffuse, la fibromyalgie primaire, la fibromyalgie secondaire, le syndrome de fibromyalgie-fibromyosite, le syndrome de fibromyosite-fibromyalgie et le rhumatisme musculaire.

4. Forme posologique pharmaceutique suivant l'une quelconque des revendications précédentes, pour une utilisation dans le traitement de la fibromyalgie ou du syndrome de fatigue chronique suivant l'une quelconque des revendications précédentes, qui est pour l'administration une fois par jour ou deux fois par jour.

5. Forme posologique pharmaceutique suivant l'une quelconque des revendications précédentes, pour une utilisation dans le traitement de la fibromyalgie ou du syndrome de fatigue chronique suivant l'une quelconque des revendications précédentes, qui est pour l'administration orale.

6. Forme posologique pharmaceutique suivant la revendication 5, pour une utilisation dans le traitement de la fibromyalgie ou du syndrome de fatigue chronique suivant la revendication 5, qui est choisie dans le groupe consistant en des comprimés, des comprimés à mâcher, des comprimés enrobés, des capsules, des granules, des gouttes, des jus et des sirops.

7. Forme posologique pharmaceutique suivant l'une quelconque des revendications précédentes, pour une utilisation dans le traitement de la fibromyalgie ou du syndrome de fatigue chronique suivant l'une quelconque des revendications précédentes, qui est pour l'administration à un mammifère, de préférence à un être humain.

8. Forme posologique pharmaceutique suivant l'une quelconque des revendications précédentes, pour une utilisation dans le traitement de la fibromyalgie ou du syndrome de fatigue chronique suivant l'une quelconque des revendications précédentes, qui est pour l'administration à un adulte.

9. Forme posologique pharmaceutique suivant la revendication 8, pour une utilisation dans le traitement de la fibromyalgie ou du syndrome de fatigue chronique suivant la revendication 8, l'adulte étant un patient âgé.
